# EUROPEAN PATENT SPECIFICATION

(11) **EP 4 170 667 B1**
(45) Date of publication and mention of the grant of the patent: **18.11.2026**
(21) Application number: 21204544.7
(22) Date of filing: 25.10.2021
(51) Int. Cl.: G16H 30/40, G16H 20/40, G16H 50/20, G16H 50/50, G16H 30/20

(54) **DATABASE-BASED CONFIGURATOR FOR THE CREATION OF ANATOMICAL MODELS**
DATENBANKBASIERTER KONFIGURATOR ZUR ERZEUGUNG VON ANATOMISCHEN MODELLEN
CONFIGURATEUR BASÉ SUR BASE DE DONNÉES POUR LA CRÉATION DE MODÈLES ANATOMIQUES

(43) Date of publication of application: 26.04.2023
(73) Proprietor: Anamos GmbH, 81541 München (DE)
(72) Inventor: Riedle, Hannah, 81541 München (DE)
(74) Representative: Stellbrink & Partner Patentanwälte mbB

(56) References cited:
- EP-A1- 3 282 380
- WO-A2-2006/000793
- US-A1- 2013 085 736

## Description

The present invention relates to a system and method for generation of tissue models, which may also be referred to as anatomical models. More particularly, the present invention relates to a database-based system for generation of digital tissue models and corresponding methods.

Tissue models, that may also be called artificial anatomical models, or simply anatomical models, represent healthy or pathologically altered body structures, such as bones, the heart, or vessels. In digital and physical (or material) form, these models can allow purely visual, or also interactive (for e.g., through realistic haptic feedback) demonstration, or serve as simulation objects. These anatomical models can thus substitute for the biological body in a variety of applications: education and training, patient education, pre-surgical planning, evaluation of treatment techniques, evaluation of medical and/or body-related products, and general demonstration purposes (e.g., functionality of a medical device).

This may be of particular advantage for education and training purposes where, in the absence of sufficiently realistic anatomical models, cadavers may need to be provided, which may limit the number of people that can be trained at any given time as the number of cadavers is limited. A number of other limitations may also arise from the use of cadavers including disposal of used cadavers, and storage of used/unused cadavers among other things. It may also not be possible to avail of cadavers representing all possible pathologies of interest to a medical trainee which may constrain the education horizon of trainees. As may be appreciated, this may also lead to a situation where a trainee may be presented with a particular pathology for the first time in a patient, for example. It may then be of interest to have a digital and/or physical (material) tissue model to practice an intended medical intervention on such a model prior to its performance on a real patient. It is an aim of the present invention to enable generation of digital and/or physical (material) tissue models with such flexibility as to allow representation of a host of pathologies.

Typically, the creation of digital anatomical models is based on a surface scan data or medical image data. Physical models may then be based on these digital models, and may be manufactured by an additive manufacturing process, for example. The medical image data may comprise, for example, X-ray data, a computer tomography scan, a positron emission tomography scan, a magnetic resonance imaging scan, or other imaging data.

Medical image data may typically comprise a plurality of features. For example, a computer tomography scan may comprise details on not only soft tissue but also hard bone tissue. It may be advantageous for modelling purposes to only model specific parts of the image data, for example, the hard bone tissue in the above example. This may be achieved by segmenting the image data, where parts of the image may be selected based on some shared property. The segmentation of 3-dimensional medical image data sets from computed tomography or magnetic resonance imaging is known. These 3-dimensional image data can be represented in 2-dimensional slice data. Each pixel of the image slices corresponds in 3 dimensions to a voxel (volume pixel) of the volume data set, to which a defined grayscale, signal or parameter value is assigned. This value may indicate a measure of the shared property based on which the image or its 3-dimensional model may be segmented. For example, it may represent a measure of hardness (or density) that may allow segmenting out the soft tissue.

During segmentation, the pixel or voxel sets are assigned to objects based on their grayscale, signal (or parameter) values, and/or their regional anatomical location. Segmentation can range from being fully manual to being fully automated and image data sets or methods can be combined, for example a segment of the hard bone tissue image with hardness above a certain threshold and a segment of the soft tissue image with hardness below a certain threshold or a combination based on grayscale value (representing density, for example) and the regional anatomical location that would enable picking out, e.g., the bony structures in a given region. The resulting objects may then be exported (for example, as surface models) for further use and/or digital post-processing. The resulting models may then depict patient-specific structures (US 2015/0250934) or represent exemplary generic variants after constructive post-processing (WO 2019/063105 A1, WO 2014/180972 A2).

Digital models can also serve as the basis for manufacturing physical models. This production may take place directly by means of additive manufacturing processes (WO 2018/222779 A1), for example, or indirectly, via casting processes using molds (WO 2019/158470 A1) and tools derived from the digital model.

While the above described solutions may be satisfactory to some extent, they have certain drawbacks and limitations. In particular, they are relatively specific as regards the input data. Normally, e.g., medical imaging data from a patient is used to generate a model, and an, e.g., idealized version of this model is generated, wherein this process is mostly a linear process. However, this is not ideal with regard to efficiency of model generation.

WO 2006/000793 A2 describes a method of creating and/or updating a model that relates to at least one measurable tissue property to at least one tissue characteristic. The method comprising: defining two or more data categories; assigning each of a plurality of training data sets to one or more of the data categories; creating and/or updating the model using only data sets categorized in a selected one of the categories.

It is an object of the present invention to overcome or at least alleviate the shortcoming and disadvantages of the prior art. In particular, it is an object of the present invention to provide a technology improving efficient generation of digital tissue models. It is a further object of the present invention to improve the realism of digital tissue models. These objects are met by embodiments of the present invention.

In a first aspect, the present invention relates to a system, wherein the system comprises a database, wherein the system is configured to, for each object of a plurality of objects, vary object parameters and thus generate parameter variations and to generate object variations, and store data relating to the parameter variations in the database, wherein the system is further configured to generate a plurality of digital tissue models, wherein generating each digital tissue model comprises combining object variations of different objects.

Each object variation may be based on a combination of the object parameters and the parameter variations of the respective object. Generating object variations may comprise post-processing.

It should be understood that the object parameters may also be referred to as object parameter values or simply parameter values. As a mere example, if the object represents an aorta, parameters may relate to a maximum extension of the aorta perpendicular to a centerline and exemplary object parameters may thus be, e.g., a list with entries 0.2 mm, 0.5 mm, 0.4 mm, 0.4 mm, 0.3 mm, 0.3 mm, 0.3 mm, 0.2 mm, 0.2 mm, 0.2 mm, where it should be understood that this is a mere example not limiting the scope of the present invention.

It should be understood that the parameter variations are varied parameters, and may therefore also be referred to as varied parameters, or variants of the parameters.

It should be understood that a combination of the object parameters and the parameter variations also encompasses that only the object parameters or only variations are used.

That is, e.g., an object with object parameters may be provided, and these object parameters are varied to arrive at variations of the object parameters. Data relating to these variations are then stored in a database. For example, the variations (i.e., the varied object parameters) as such may be stored in the database. Additionally, or alternatively, rules relating to the parameter variations may be stored in the database.

Based on the object parameters and the parameter variations, a plurality of different object variations can then be generated. Object variations may also be generated by post-processing any of the objects or object variations (derived by combining object parameters and their variations). For example, starting with a first object and its object parameters, a first object variation may be generated by varying at least one of the object parameters. Then, a post-processing of the first object variation may be carried out to generate a second object variation. The post-processing may involve any of a plurality of operators such as a union, an intersection, a difference with another object or object variation, remeshing, smoothing, cropping, addition of radii, or any other operator (that may not involve varying any of the object parameters of the first object).

As regards the object variation of an object, the skilled person will understand that also the object as such can be considered to constitute such an object variation, i.e., the "identity" variation of the object, i.e., the variation of the object where the object remains unchanged. However, it should be understood that the term "object variation" also includes variations of the object leading to an object variation different from to the original object, i.e., different from the object the object variation is based on. Such a variation may also be referred to as non-identity variation. Thus, when reference is herein made to "object or object variation", it should be understood that either an object as such, or a non-identity variation of the object may be used.

By combining object variations of different objects, a plurality of different tissue models may be generated.

Any of the above processes, e.g., combining with another object or object variation, post-processing, or constructive editing, may also be carried out on combinations of objects and/or object variations. For example, an object may comprise a digital model for a left ventricle that may be obtained from medical imaging data. An object variation may comprise a digital model for a variation of right ventricle with a smaller volume than the object used to derive it. The digital models of the left ventricle and the right ventricle with the smaller volume may then be combined, by a union operator, for example. This new model comprising the left and right ventricles may then be further post-processed, constructively edited, or combined with other digital models using any of the operators described here.

Thus, the present technology provides a system efficiently generating a plurality of different tissue models based on the object parameters and their variations, and object variations, thereby providing a technology with great efficiency as regard the creation of a plurality of digital tissue models.

The system may further be configured to parametrize an object and thus generate the object parameters, and store the object parameters in the database. The object parameters may comprise parameters describing the anatomical features of the object, such as the location of the center line and a maximum extension perpendicular to the central line at each of those locations for an aorta as described above. They may also include, however, the locations (corresponding to voxels, for example) of the different parts of the aorta.

The system may also be configured to generate an object before parametrizing it.

The system may be configured to generate an object from surface scan data or medical imaging data. For example, if the object relates to an inner shape of an aorta, the object may be generated from medical imaging data showing the aorta.

The system may alternatively or additionally be configured to generate an object based on an original object. In the above example, the object may also relate to an outer shape of the aorta. If, e.g., only the inner shape (i.e., the inner lumen) can be obtained by medical imaging, the inner shape would be the original object, which may also be referred to as the native object. However, the outer shape of the aorta can be generated (e.g., derived) based on the original object. For example, one may assume that the aorta has a wall thickness 1 mm and one may thus derive the shape of the outer shape based on the original object (being indicative of the inner shape) and the wall thickness. Thus, objects may also be generated based on an original object, wherein the original object is generated from medical imaging data, for example.

Generally, objects may be additionally post-processed and/or constructively edited to create object variations. Such object variations may then also be parametrized and varied to generate further object variations. Each such object or object variation may thus have its own set of parameters and variants and will later e.g., for the creation of the heart, be combined differently with other objects or object variations of other objects.

Each object is indicative of a tissue. For example, it may be indicative of a hard tissue, such as bone, or a soft tissue, such as the ventricles of a heart. It may be indicative of either human or animal tissues. Embodiments of the present invention may be of particular advantage in generating a range of digital tissue models representing various pathologies, some of which may be generated for significantly pedagogical purposes as well.

At least one object is a surface in three-dimensional space. In case the object is indicative of a tissue, the surface in three-dimensional space may provide an accurate relative positioning of different parts of the tissue, even if the absolute dimensions do not correspond to an actual tissue. For example, an object representing a human heart in three-dimensional space may comprise atria, ventricles, and the aorta all in their correct relative position as in a human. By suitably modifying the dimensions of the three-dimensional model, the heart of a child may then be generated, for example.

The data relating to the parameter variations may comprise the parameter variations. This includes the actual variations resulting from varying the values of object parameters. For example, for a blood vessel with an object parameter corresponding to a thickness of the vessel wall, the variations may comprise a thickness of, say, 1 mm and 2 mm. Then the data relating to the variations may comprise the actual objects representing the blood vessel with walls of thickness 1 mm and 2 mm respectively. It may also just comprise the list of values 1 mm and 2 mm for the wall thickness for this example.

The data relating to the parameter variations may comprise rules for generating the parameter variations. This may include, for example, a rule comprising scaling by a constant factor, which may be applied to scale a given parameter by the constant factor. Thus, this may be used to more generally define different variations that may be generated.

The system described above may be configured to store the object and/or the object variations in the database. This may be advantageous in allowing the object and/or its variations to be recovered at a later time and modified, for example, to generate a different digital tissue model from the one generated before the modification. Thus, embodiments of the present invention may allow the process of generating digital tissue models from medical imaging data to be non-linear as several digital tissue models may be generated by appropriate modification of objects and/or their variations stored in the database.

The system may be further configured to store the plurality of different digital tissue models in the database.

The system as above may comprise a processor and a memory.

The memory may comprise the database.

The system may further comprise a display, and the system may be configured to display the different digital tissue models on the display.

The system may be configured to generate a plurality of different material tissue models, wherein each of the material tissue models corresponds to a digital tissue model. The material tissue models may be of advantage in preparing for an upcoming medical intervention or for educational purposes, for example.

The system may be configured to generate the plurality of different material tissue models by additive manufacturing. This may be achieved by 3D printing the digital tissue models, for example.

The system may be configured to generate the plurality of different material tissue models by a casting process. This may allow the material tissue models to be manufactured using materials which may not be suitable to be used in an additive manufacturing process. Depending on the choice of materials to be used for the material tissue models, a casting or additive manufacturing process may be thus used.

The casting process may use molds and tools based on the digital tissue model.

The system is further configured to store a set of operators, and the object variations are combined using any of the operators in the set of operators to generate the digital tissue model. The use of operators may be particularly suitable for embodiments of the present technology as different digital tissue models may be assembled together using the operators to generate digital tissue models for a variety of tissues, possibly with specific variations for a particular component. For example, a digital tissue model for a human heart may be assembled by combining object variations for the ventricles, the atria, and the aorta. Such an object variation may be used to represent a particular pathology of the left atrium, for example. The digital tissue model for the heart may then be representative of a heart with the pathological left atrium that may be useful when planning a medical intervention to correct the pathology, for example.

The object parameters and parameter variations may be combined to generate the object variations by means of any of the operators in the set of operators. Generating object variations may further comprise any of constructively editing, post-processing, or combining with an object or object variation by means of an operator in the set of operators. For example, a remesh after a Boolean operation may be used. As another example, a first object may be parameterized and the values of at least one of its parameters varied to generate a first object variation. Then, this first object variation may be combined with a second object or a second object variation by means of an operator in the set of operators. Any of the first object and the second object and their corresponding object variations may be further post-processed or constructively edited before combining them. The resulting combination of the first and second object or variations thereof may then be further post-processed and/or constructively edited and/or combined with a third object or an object variation thereof.

The set of operators may be stored in the database. They may, thus, represent the set of operations that may be performed to combine any of the objects or object variations that may also have been stored in the database.

The set of operators may comprise a difference operator. The difference operator may be used, for example, to generate hollow digital tissue models from corresponding filled ones. For example, a digital tissue model for a hollow blood vessel may be generated by differencing an object variation representing the inner volume of a blood vessel from one representing the total volume of a blood vessel.

The set of operators may comprise a union operator. The union operator may be used, for example, to generate the human heart model as described above, by taking a union of the object variations corresponding to the atria, the ventricles, and the aorta.

The set of operators may comprise an intersection operator.

The system may be further configured to store data relating to allowed combinations of object parameters, parameter variations, and/or object variations. For example, for the case of a blood vessel, it may not be possible to combine a variation with an inner volume of 2 mL with a variation comprising a total volume of 1.5 mL.

The data relating to allowed combinations may comprise a list of allowed combinations of object parameters, parameter variations, and/or object variations. For example, the particular combination of inner volume being 1.5 mL and total volume being 2 mL as such may comprise the data relating to allowed combinations of object parameters and variations.

The data relating to allowed combinations of object parameters and variations may comprise rules for allowed combinations of object parameters and variations. The data relating to allowed combinations of object parameters and variations may also include the rule that a variation with inner volume of certain value may not be combined with a variation with a total volume of a certain value if the value of the inner volume is larger than the value of the total volume. This may, thus, allow including a set of general rules about allowed combinations.

The system may be further configured to store a set of constructive elements. The constructive elements may be used to allow the digital tissue models generated using embodiments of the present technology to be integrated into a different simulation environment, for example. They may include, for example, adapters to use a digital tissue model for a blood vessel together with a simulation depicting flow of blood through the vessel.

The system may be further configured to generate constructive element objects based on the constructive elements and to parameterize the constructive element objects to thus generate constructive element parameters, and to vary the constructive element parameters and thus generate constructive variations. The constructive elements may themselves be parameterized. For example, in case of a blood vessel and its integration into a simulation of blood flow described above, it may be of interest to simulate blood flow through a vessel with thicker walls. In this case, the dimensions of the adapters described above may also be varied to integrate the blood vessel into the simulation environment.

The system may be further configured to store data relating to the constructive variations.

The system may be configured to store data relating to the constructive variations in the database.

Any of the plurality of different digital tissue models may be generated based also on a combination with the constructive element parameters and the constructive variations.

Combination with the constructive element parameters and the constructive variations may further be achieved by means of any of the operators in the set of operators described above.

Any of the plurality of different digital tissue models may also be configured to be used together with a simulation environment.

The set of constructive elements as described above may comprise adapters to allow the digital tissue model to be integrated into the simulation environment.

The system may further comprise a user interface configured to at least accept input from a user. The user interface may help to allow further individualization of the digital tissue models generated. For example, it may comprise a functionality allowing a user to choose an operator corresponding to scaling by a constant factor and prompting the user to input or choose from a list the constant factor.

The user interface may comprise the display.

Each object as described above may comprise a surface and/or volume mesh comprising at least two nodes, and a number of edges between any two nodes. This may allow using any mesh operations to further individualize any of the digital tissue models. In particular, it may make combination using the operators as described above easier as each of the objects may be stored as a set comprising the nodes and the list of edges between them. Then a union operation, for example, may be simply achieved by the union of the sets of nodes and edges. It will be understood that also the tissue models or the object variations may each comprise a surface and/or volume mesh comprising at least two nodes and a number of edges between any two nodes.

The object parameters as described above may comprise locations of the nodes and a list of edges between the nodes. This may represent an extensive list of parameters such that these may be used to characterize any other features of the object such as its total volume, or its inner volume.

The number of edges between any two nodes may be at least 0. This may be of particular advantage to correct for artefacts arising from generating objects from medical imaging data. For example, when generating an object corresponding to, e.g., a human skull from an X-ray image, a segmentation may be carried out based on the grayscale values being above a certain threshold. There may be noise pixels in the image with a grayscale value above the threshold which would then be part of the object. In this case, because they have 0 edges connected to them, they may be deleted simply.

At least one of the nodes as described above may be configured to be moved based on an input from the user. This may allow a user to configure a digital tissue model individually, before manufacturing a material tissue model for it, for example.

The system may be further configured to not allow at least one of the nodes to be moved. This may help prevent changing, for example, a basic structure of the tissue represented by the digital tissue model. For example, the system may be configured to allow only a scaling of the entire model such that all the nodes move in a shape-preserving manner, and movement of any individual node may not be possible.

The system as described above may be further configured to allow the lengths of any of the edges to be changed. For example, for scaling by a constant factor, the lengths of all the edges may be scaled by the constant factor.

The system may be further configured to allow adding at least one node. This may be of particular advantage in re-meshing the digital tissue model. By adding more nodes, for example, it may then be possible to improve the resolution of features represented by the digital tissue model.

The system may be further configured to allow adding or deleting an edge between any two nodes.

The system may be configured to allow deletion of any of the nodes and all the edges connected to the deleted nodes. When generating an object from a medical image, for example, artefacts such as an island (corresponding to a set of nodes connected to each other but not to any other nodes in the model) may emerge in the object. These islands may be deleted before further processing of the object is carried out.

The object parameters comprise parameters describing the geometry of the object. For example, for a blood vessel, the object parameters may comprise a parameter defining the shape of the blood vessel, such as a circle, and its diameter at various points along the length of the blood vessel.

The system as described above may be configured to assign manufacturing parameters to control a manufacturing process of the material tissue models. Manufacturing parameters may comprise parameters controlling the material to be used for the material tissue models, the surface roughness, etc.

The input from the user as described above may comprise any of a choice of parameters, or a choice of variations. A choice of parameters may comprise choosing the actual parameters to be varied. For example, for a blood vessel, this may correspond to choosing the total volume from among a choice of total volume and inner volume. This may lead to a digital tissue model with the same inner volume but a different total volume, representing a different wall thickness, for example. A choice of variations may comprise choosing values for the parameters chosen above. For example, it may comprise choosing a total volume of 2 mL from among a choice of 1.5 mL and 2 mL.

The input from the user may further comprise a choice of operators.

The input from the user may comprise a choice of constructive elements. The choice of constructive elements may depend on, for example, the simulation environment intended to be used with the digital tissue model.

The input from the user may comprise a choice of manufacturing parameters. The choice of manufacturing parameters may depend, for example, on the pathology to be represented by the material tissue model. For the material tissue model of a blood vessel of a patient suffering from atherosclerosis, for example, a less elastic material may be chosen than for a healthy blood vessel.

The system may be further configured to store data comprising constraints on the allowed object parameter values for an object corresponding to a digital tissue model.

The system may be configured to apply boundary conditions limiting the varying when varying the object parameters. For example, an inner volume of a human blood vessel may be limited by the total volume of blood in the human body, i.e., less than, e.g., 5 L.

The boundary conditions for varying a first object parameter may depend on a second object parameter or a variation of the second object parameter from either the same or another object. For example, for a blood vessel, once a total volume has been fixed, the inner volume may not be allowed to take on a value larger than the total volume.

It should be understood that applying boundary conditions may also be independent from the database, storing of data relating to the variations in the database and generating a plurality of different tissue models. That is, embodiments of the present invention also relate to a system configured to generate digital tissue models, wherein the system is configured to vary object parameters and thus generate variations, wherein the system is configured to apply boundary conditions limiting the varying when varying the object parameters. However, as described, applying the boundary conditions may also be used together with the other features of the first aspect.

The system may be a computer implemented system.

The system may comprise a first data processing unit configured to vary the object parameters and thus generate the parameter variations, and to store the data relating to the parameter variations, and a second data processing unit configured to generate the plurality of different digital tissue models, wherein generating each digital tissue model comprises combining the object variations of different objects. That is, the different functionalities may be distributed between different data processing units. It may thus be possible, e.g., to have a first data processing unit with higher performance performing the first functionalities (which may be associated with a lot of computational power), while one functionality not associated with such a high computational power may be accomplished by a data processing unit with lower computational performance.

The system may be configured to generate the plurality of different digital tissue models, wherein generating each digital tissue comprises combining object variations of different objects, with a time delay of at least 1 hour, preferably at least 24 hours, after storing the data relating to the parameter variations in the database. In particular when the different functionalities are distributed among different data processing units, they may also be performed at substantially different times. In particular, functionalities requiring a high computational power (and potentially more computational time) may be pre-performed, and functionalities requiring lower computational power (and potentially less computational time) may be performed when needed.

In a second aspect the present invention relates to a method to generate digital tissue models, wherein the method comprises using a system comprising a database, and wherein the method further comprises, for each object of a plurality of objects, varying object parameters and thus generating parameter variations and generating object variations, and storing data relating to the parameter variations in the database, wherein the method further comprises generating a plurality of digital tissue models, wherein generating each digital tissue model comprises combining object variations of different objects.

The method may further comprise parameterizing an object and thus generating the object parameters, and storing the object parameters in the database.

The method may further comprise generating an object.

The object may be generated from surface scan data or from medical imaging data.

Generating the object from surface scan data or from medical imaging data may comprise segmenting images from the surface scan data or the medical imaging data based on a shared property of different parts of the image and either including or excluding parts of the image based on the shared property.

The shared property may comprise any of a grayscale value, a signal or parameter value, or a regional anatomical location. For example, objects may be generated for parts of the image with a grayscale value above a certain threshold value in an X-ray. This may represent segmenting out regions with a density higher than a threshold density, corresponding to bones, for example.

Generating the object may comprise generating a three-dimensional representation of the segmented image.

An object may also be generated based on an original object.

Parameterizing the object may comprise generating a set of parameters characterizing anatomical features of the object. This may comprise generating parameters such as volumes, surface areas, shapes, and diameters. It may also comprise generating a mesh of nodes and a list of edges connecting any two nodes.

Generating parameter variations may comprise choosing values for any of the parameters in the set of parameters characterizing at least one anatomical feature of the object. The values chosen may comprise, for example, a representative set of different pathologies in the anatomy of the tissue being digitally modelled. This may allow fast and efficient recovery of such pathologies for further use.

The method comprises using a system as described above, wherein generating a plurality of different digital tissue models comprises using any of the operators in the set of operators to combine the object variations.

The method may comprise further processing any of the plurality of different digital tissue models.

Further processing of any of the plurality of different digital tissue models may comprise any of a point-based, surface-based, or volume-based processing. In particular, it may include a smoothing of the surfaces, for example. This may be of particular advantage when a material tissue model is to be manufactured from a digital tissue model.

The method may further comprise individualizing any of the plurality of different digital tissue models.

Individualizing any of the plurality of different digital tissue models may comprise adapting the geometry of a digital tissue model.

Adapting the geometry of a digital tissue model may comprise choosing values for parameters characterizing additional geometric features of the object.

The method may comprise using a system as described above, wherein adapting the geometry of the digital tissue model may comprise moving individual nodes of the surface or volume mesh of the digital tissue model.

The method may comprise using a system as described above, and wherein the method may further comprise generating the plurality of different material tissue models.

The method may further comprise assigning a material and/or material properties to manufacture at least part of the material tissue model corresponding to a digital tissue model.

The method may further comprise assigning manufacturing parameters to control the manufacturing of the material tissue model.

The manufacturing parameters may comprise any of a manufacturing direction, tolerance, surface roughness, color, coating, grinding, curing, among others. For example, tolerance may be of particular relevance when the digital tissue model represents tissue corresponding to a region of the human brain. It may then be of advantage to have a material tissue model significantly accurate to 1 mm, for example.

When varying the object parameters, boundary conditions may be present limiting the varying.

The boundary conditions for varying a first object parameter may depend on a second object parameter from either the same or another object or a variation of the second object parameter.

It should be understood that applying boundary conditions may also be independent from the database, storing of data relating to the variations in the database and generating a plurality of different tissue models. That is, embodiments of the present invention also relate to a method to generate digital tissue models, wherein the method comprises varying object parameters and thus generating variations, wherein when varying the object parameters, boundary conditions are present limiting the varying. However, as described, applying the boundary conditions may also be used together with the other features of the second aspect.

The method uses the system as described above.

The method may be a computer implemented method.

The system may comprise a first data processing unit and a second data processing unit, and varying the object parameters and thus generating variations generating parameter variations and generating object variations and storing the data relating to the variations in the database may be performed by the first data processing unit, and generating the plurality of different digital tissue models, wherein generating each digital tissue comprises combining object variations of different objects, may be performed by the second data processing unit.

There may be a time delay of at least 1 hour, preferably at least 24 hours, between storing the data storing data relating to the object variations in the database and generating the plurality of different digital tissue models, wherein generating each digital tissue model comprises combining the object variations of different objects.

The system may be configured to perform the method as described above.

In a third aspect, the present invention relates to a computer program product configured, when run on a digital processing unit of a system, to perform the method according to any of the preceding method embodiments.

In a fourth aspect, the present invention relates to a use of the system as described above to perform the method as described above.

That is, overall, embodiments of the present invention are directed to a computer-implemented method for creating digital and physical anatomical models. This model can, for example, be of a human heart, but the method is not limited to any anatomy or species. The models generated by embodiments of the present invention are generic variants that are derived from an image data set of an original biological human or animal, for e.g., a patient. The generic approach allows constructive modifications of specific features of the biological original in the creation process, making it not specific to that one original source (e.g., patient) any more. Furthermore, embodiments of the present invention also relate to a database-based configuration of the generated models to scale the linear modelling processes to a flexible, more efficient process. A system for carrying out embodiments of the present invention as described below may be called a database-based anatomical configurator.

It will be understood that embodiments of the present invention therefore also relate to post-processing of the object, e.g., to generate models representing the variants. Post-processing may allow the deformation and transition of the exported models into any shape, and therefore any variation of the anatomy, which may be more advantageous than having patient-specific models. Since the models are thus not based on data of a specific anatomy or pathology, a plurality of options (models) may be derived. These may have a number of applications including medical training. However, they may not be useful for patient-specific pre-surgical planning. One advantage of this generic approach may therefore be independence from specific source data and the possibility to create any anatomy or more importantly any pathology.

Embodiments of the present invention thus relate to a generic approach making it possible to efficiently derive a large number of models, including variants modified from the original structure, e.g., from just one data set. This may be a further improvement over prior processes. The creation of digital models (patient-specific or generic) is typically a linear process, i.e., the number of models created grows linearly with the number of image data sets available. Patient-specific modelling may typically be time consuming and inefficient, since the process is based on one specific data set and typically creates models in that specific variation. However, with the presently described approach, very few design constraints are given, and the process may still be linear, since each variation is created individually. Thus, embodiments of the present invention provide a generic approach to efficiently generate a plurality of different digital tissue models.

Embodiments of the present invention will now be described with reference to the drawings, which should only exemplify, but not limit, the scope of the present invention.
- Fig. 1: depicts an object derived from medical imaging data according to embodiments of the present invention,
- Fig. 2: depicts a flow chart of steps used in embodiments of the present invention,
- Fig. 3: depicts another flow charts of steps used in embodiments of the present invention,
- Fig. 4: depicts medical imaging data and objects derived from the medical imaging data according to embodiments of the present invention,
- Fig. 5: visualizes the generation of an object relating to a total aortic volume according to embodiments of the present invention, and
- Fig. 6: depicts a digital heart model generated by embodiments of the present invention.

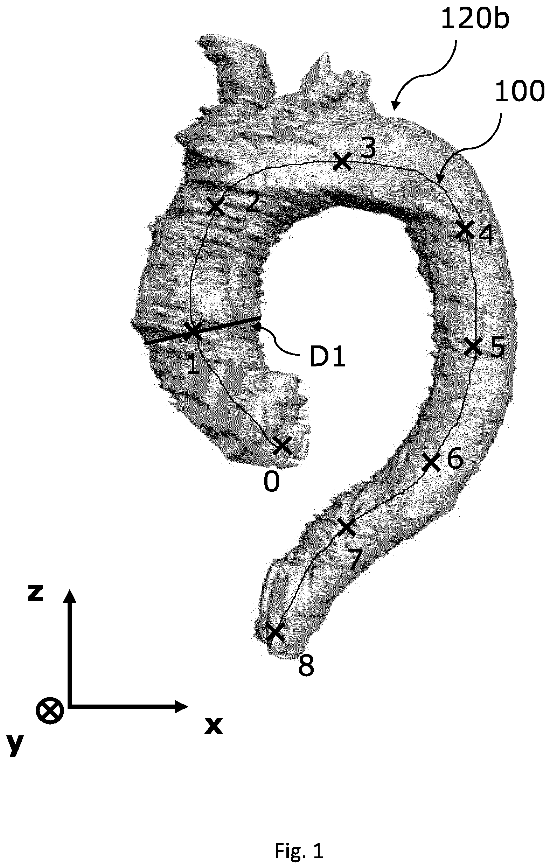

A first example of an embodiment of the present technology will be described with reference to Fig. 1. As described above, embodiments of the present technology use medical imaging data, e.g., CT data. For example, such medical imaging data may depict a greyscale image of an aorta. Based thereon, a virtual object (which may also be referred to as a digital object), e.g., including the inner extension of, e.g., an aorta, may be generated. An initial digital object generated from the scan data and/or from the medical imaging data may also be referred to as a native object.

Fig. 1 depicts a representation of such a virtual object to which additional annotations have been added for explaining embodiments of the present technology.

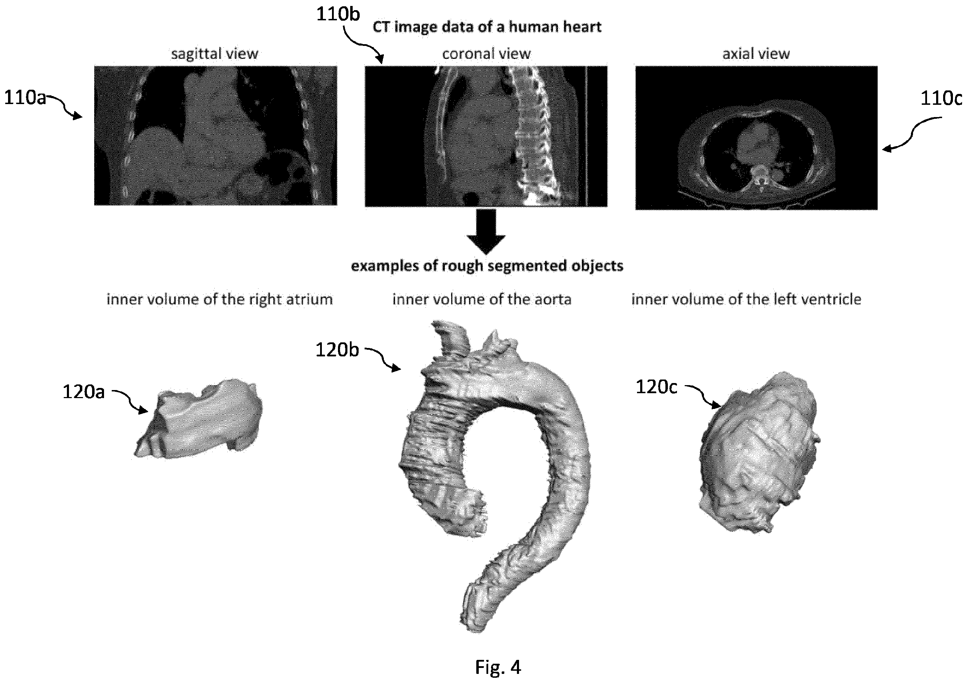

For example, a centerline 100 is depicted in Fig. 1 indicating the center of the inner extension of the aorta 120b (shown in Fig. 4), which is an example of a blood vessel.

The object (i.e., inner extension of the aorta 120b) may be parameterized, i.e., described by means of parameters, which may also be referred to as parameter values.

In the described example, where the object is the inner extension of aorta 120b, i.e., a blood vessel, one parameter may be the location of the centerline 100. This location may be identified, e.g., according to the below table 1.

**Table 1**

| **Point on centerline** | **x/mm** | **y/mm** | **z/mm** |
|---|---|---|---|
| 0 | 0 | 0 | 0 |
| 1 | -0.5 | 0 | 0.9 |
| 2 | -0.4 | 0.4 | 1.8 |
| 3 | 0.3 | -0.3 | 2 |
| 4 | 0.7 | -1.2 | 1.8 |
| 5 | 0.7 | -0.8 | 0.9 |
| 6 | 0.6 | -0.4 | 0 |
| 7 | 0.2 | -1.2 | -0.4 |
| 8 | -0.1 | -0.6 | -1.1 |

That is, according to the above table, parameters may relate to the location of the centerline (i.e., its x, y, and z coordinate). In the above, 9 such points have been identified, with each point being distanced from the neighboring points by 1 mm. However, it should be understood that this is merely exemplary and that also another number of points (relating to the binning or sampling rate) may be used.

Other parameters that may be derived from the object may be, e.g., parameters relating to the size of the object. Continuing with the above example, e.g., the maximum diameter corresponding to the respective point on the centerline (and perpendicular to the centerline) may be such a parameter, and exemplary values are included in the below table 2.

**Table 2**

| **Point on centerline** | **Maximum inner diameter /mm** |
|---|---|
| 0 | 0.2 |
| 1 | 0.5 |
| 2 | 0.4 |
| 3 | 0.4 |
| 4 | 0.3 |
| 5 | 0.3 |
| 6 | 0.3 |
| 7 | 0.2 |
| 8 | 0.2 |

In Fig. 1, the maximum inner diameter D1 for point 1 in the centerline has been identified as D1.

It will be understood that the above is merely exemplary and that a multitude of parameters may be generated when parametrizing the object.

In a next step, the object may be varied, i.e., the parameters may be varied, to thus generate variants of the object that may be called object variations.

Continuing with the above example, e.g., the complete object (or parts thereof) may be scaled. For example, the length of the inner extension of aorta 120b may be maintained (and thus, the location of the centerline and the data provided in table 1 may be maintained), while scaling the extension of the inner extension of aorta 120b perpendicular to its center line. E.g., when scaling the aorta 120b by a factor of 1.5, one would arrive at a maximum inner diameter of this variation according to table 3.

**Table 3**

| **Point on centerline** | **Maximum inner diameter /mm** |
|---|---|
| 0 | 0.3 |
| 1 | 0.8 |
| 2 | 0.6 |
| 3 | 0.6 |
| 4 | 0.5 |
| 5 | 0.5 |
| 6 | 0.5 |
| 7 | 0.3 |
| 8 | 0.3 |

Thus, an object variant or object variation may be created.

The present technology may also store at least part of the information described above in a database. More particularly, the virtual object (which may also be referred to as digital object), the parameters (e.g., the locations of the centerline as in table 1 and the maximum inner diameter as in table 2) and data relating to the variations may be stored. The data relating to the variations may be rules for generating the variations (e.g., "multiply the values for the inner diameter by 1.5") or the variations as such, i.e., the varied parameters (e.g., above table 3).

As discussed, Fig. 1 depicts an object representing an inner extension of an aorta.

In embodiments of the present technology, a plurality of such objects may be used. For example, based on the object representing an inner extension of the aorta, another object may be generated representing an outer extension of the aorta.

For example, such a second object representing the outer extension of the aorta may be generated based on the object representing the inner extension of the aorta and assuming a constant wall thickness, to thus arrive at a second object representing the outer extension of the aorta.

Again, the second object may be varied to create variations of the second object, e.g., by scaling the diameters of the second object.

Thus, a plurality of different objects (e.g., representing the inner diameter of the aorta, and the outer diameter of the aorta) may be generated, and for each object, a plurality of variations may be generated. In this regard, it is noted that also the object as such (e.g., the native object derived from the imaging data) will be referred to as a variation, where the skilled person will understand that this an "identity" variation, i.e., a variation where the object is not changed.

In embodiments of the present technology, the variations of different objects are combined. Continuing with the above example, the object representing the inner diameter of the aorta may be identified as V(0,1), i.e., the 0-variation of the first object (which may also be referred to as the identity variation of the first object), the first variation of the inner diameter of the aorta may be identified by V(1,1), etc. Correspondingly, the object representing the outer diameter of the aorta may be identified as V(0,2), i.e., the zero-variation of the second object, the first variation of the outer diameter may be identified as V(1,2), etc.

Variations of different objects may be combined in embodiments of the present technology to thus generate a digital tissue model. As a mere example, the 5th variation of the first object, i.e., V(5,1) may be combined with the fourth variation of the second object, i.e., V(4,2).

It will be understood that such a combination may result, e.g., in data representing both the inner and outer extension of the aorta. Furthermore, certain rules may apply as regards which combinations may be performed. Continuing with the above example, e.g., there may be the requirement that at every point, the inner extension represented by V(n,1) is smaller than the outer diameter V(m,2), and only combinations of variations fulfilling this requirement can be generated.

Thus, overall, embodiments of the present technology relate to generating object variations and combining variations of different objects to thus arrive at a plurality of different tissue models.

While in the above described example, the object (and their variations) related to inner and outer extensions of the aorta, it will be understood that this example was discussed for simplicity only, and that the object and their variations may also relate to more complex aspects, e.g., different parts of the heart and the aorta.

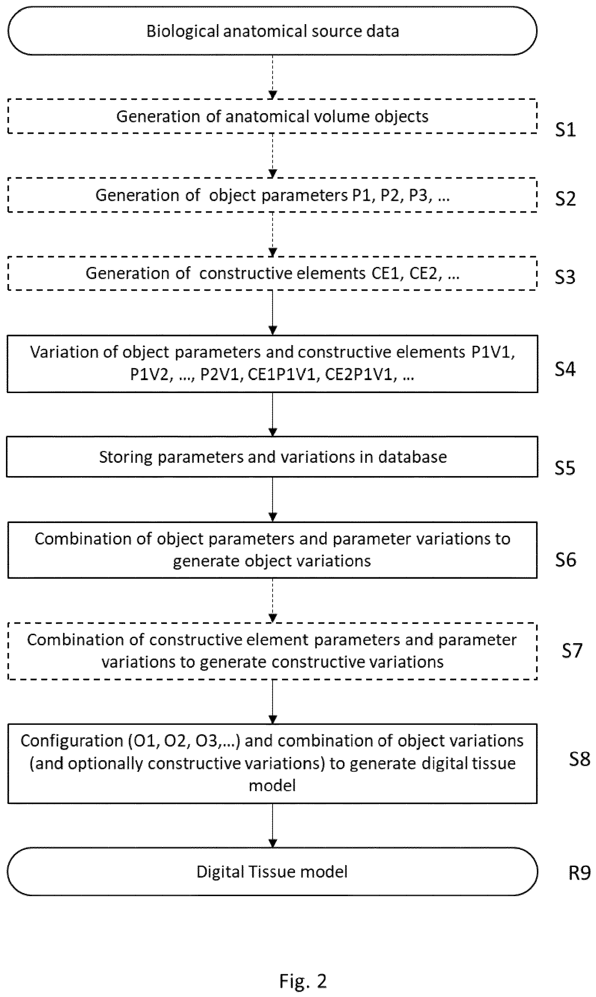

Fig. 2 depicts an exemplary flow chart of the different steps that may be employed to realize embodiments of the present invention. As depicted, the method may comprise, for example, 8 steps, S1 to S8. However, it should be understood that in embodiments of the present technology, some of the steps may also be omitted.

In a first step, labelled as S1, one or more volume or surface objects may be digitally created from surface scan data or medical image data of a biological anatomical source (human or animal) - in the example discussed before with reference to Fig. 1, the object may be the inner dimensions of the aorta 120b. This may be accomplished by using segmentation and surface mesh post-processing methods, for example. Segmentation may refer to manual to fully automated image processing in which voxels are accumulated based on their grayscale, signal or parameter values, and/or their regional anatomical location. For example, anatomical components may be represented as vertices of a graph and an edge between two vertices may represent components that may be included together based on their spatial, anatomical proximity, such as the heart, the lungs, and the rib cage. Alternatively, vertices may be connected based on their connectivity in carrying out a process, such as the mouth, throat, esophagus, and stomach in carrying out digestion.

Based on a segmentation, one or more digital objects (e.g., the 3-dimensional surface indicating the inner dimensions of an aorta) may be created that includes parts of the image that have been accumulated. The created objects may directly or indirectly approximate geometrical anatomical volumes and their relative and absolute positioning. For example, these digital objects can be the geometry of the inner or outer volume of a vessel (as discussed in conjunction with Fig. 1), the geometry of the total volume of the trachea or the geometry of the volume of a bone. The segmented objects may then be exported as net, surface or volume models. It will be understood that the objects may be generated directly from surface scan data, but that they can also be derived, e.g., from other objects. In the example described above, one object (representing the inner diameter of the aorta) was generated directly from medical imaging data, while another object (representing the outer diameter of the aorta) was derived based on the first object.

The segmented digital objects generated in the previous step may be additionally post-processed and/or constructively edited. As discussed, new objects can be created by post-processing and/or constructively editing and/or constructively combining other objects. Then, in a second step, labelled S2, the digital objects may be parametrized. A set of parameters, for example k parameters, P1 to Pk, may be generated to characterize the digital objects. These parameters may represent the sum of geometrical characteristics describing an anatomical region, and therefore, object. For example, the object describing the inner volume of a blood vessel may comprise parameters characterizing e.g., its position or shape. The parameters may also be referred to as parameter values. In the example described above with reference to Fig. 1, the parameters were the values for the location of the centerline of the inner extension of the aorta and the values for the maximum inner diameter, but it should be understood that these parameters were merely exemplary.

Any of the k parameters may be varied to generate a set of, for example, m parameter variants, V1 to Vm, respectively (see step S4).

As described above, the parameters P1 to Pk may describe the geometrical characteristics of a digital object. For example, for a blood vessel, the inner volume could have the parameters, P1, shape of its cross section, P2, its diameter, and P3, the distribution of the cross section and/or diameter along the vessel. As may be appreciated, the number of parameters may preferably comprise an exhaustive list used to describe a particular characteristic. For instance, the inner volume may comprise another parameter P4 and P1 may be an ellipse in the example above. In this case, P2 may represent an average diameter, whereas the parameter P4 may represent a length of the minor axis of the ellipse, for example. Knowing P2 and P4, the full shape of the ellipse may thus be described. Alternatively, when P1 is a circle, for example, P2 and P4 may be chosen equal.

As another example, one may consider the outer or total volume of the blood vessel. The parameters of the total volume of the blood vessel may be similar to the inner volume, but may further comprise parameters defining for example the structure of the vascular wall.

By setting different values for the parameters, P1 to Pk, a set of parameter variants, V1 to Vm, may be created (see again step S4). These values may be based on the original biological source data and/or a healthy variety and/or a pathological variety and/or may be influenced by the intended application. E.g., the digital anatomical model may be created for manufacturing a physical anatomical model, using additive manufacturing for example, and manufacturing requirements may request certain variants, which might differ from the biological original anatomy. For example, one such variant might be one where the physical (material) anatomical model may need to have a minimum wall thickness, e.g., in order to fulfill the manufacturing specifications.

Once the parameters characterizing a digital object have been generated, a step S3 may follow, corresponding to generation of, for example, constructive elements, characterized by the parameters CE1 to CEr. In embodiments, this step may be absent. That is, the skilled person will understand that this step is an optional step. In other words, in addition to the parameters P1 to Pk, for example, an anatomical model may also include constructive elements parameters, CE1 to CEr. These constructive elements, may not be derived from medical image data, but instead, e.g., be provided based on a possible use of the anatomical model. For example, the (digital) anatomical model may be used in a simulation environment. In this case, the constructive elements, characterized by the parameters CE1 to CEr, may represent connectors to the simulation environment comprising, for example, a pump simulation of blood flow through a vessel. In this case, the constructive elements may include, for example, adapters at the end of the modeled vessel to allow a connection to the pump. These constructive elements may be created using different software tools, including CAD (computer-aided design) systems, and depending on the element may also be parameterized similar to the anatomical features. Thus, this may allow the generation of variants similar to the parameters P1 to Pk.

In step S4, different variations of the parameters, P1 to Pk, and the constructive elements' parameters, CE1 to CEr, may be generated. These are depicted in Fig. 2 as P1V1 (corresponding to a first variant V1 of a parameter P1), P1V2 (corresponding to a second variant V2 of the parameter P1), P2V1 (corresponding to a first variant V1 of the parameter P2), and so on. Again, with reference to the example described above with reference to Fig. 1, a first variant of a first parameter (i.e., P1V1) may be that the maximum inner diameter at a point 0 on the centerline is 0.3 mm (corresponding to line 1 in table 3) and correspondingly P2V1 would be 0.8 mm (corresponding to line 2 in table 3). These variations may then be stored in a database in a subsequent step, S5. As discussed before, both the parameters and data relating to the variations may be stored in the database. The data relating to the variations may be the values of the variations and/or rules for generating the variations, as described before.

By variable combination of different variants of the parameters, P1 to Pk different object variations can be derived, in a further step S6. A particular choice of the parameters, P1 to Pk, (for example, a choice comprising only the parameters of the internal volume, P1 to P4, as described above), with a corresponding set of parameter and variations values, may then represent a variation of the object, which may also be called object variation. A plurality of object variations may be generated following in step S6.

By a variable combination of different variants of the constructive elements' parameters, CE1 to CEr, different variations of the constructive elements can be derived, in an optional further step S7.

A plurality of object variations and optionally constructive element variations may then be combined in a further step S8, using any operator from a set of operators as described above, to obtain a plurality of different digital tissue models. In other words, the anatomical model generated in step S8 and output as result R9 (which may also be referred to as tissue model) is a combination of object variations, wherein the object variations are based, e.g., on varying parameters of the objects and optionally constructive element parameters. An exemplary object variation may be, for example, (P1, P2V1, P3V1, P4, P5, P6, ..., Pk). That is, the anatomical model may comprise an object variation corresponding to a first original object (P1, ..., Pk), except for entries 2 and 3, where the first variation P2V1, P3V1 of parameters P2 and P3 is used. The anatomical model may further comprise another object variation corresponding to a second original object with the parameter variation (P1V1, P2, P3, ...., Pm), for example, where the second original object may be parameterized only by m parameters. Corresponding considerations also apply to the constructive elements and their variations.

The combination of the parameters, P1 to Pk, and possibly constructive elements' parameters, CE1 to CEr, to generate an object variation and possibly constructive element variation, and the combination of those variations to generate a digital tissue model, may be done via operators, O1 to Oj, in a model-specific order. It may be preferable to use only one variant (including the original parameter values corresponding to the object derived from medical imaging data) of each parameter, P1 to Pn, and/or constructive elements' parameters, CE1 to CEr, to generate an object variation and/or constructive element variation.

In embodiments, it may be possible that some variants of one set of parameters, say P1 to Pi, may only be combined with specific variants of another set of parameters, say Pi+1 to Pk. This may be the case when anatomical models may be constructed based on a specific variant of that other parameter. For example, a hollow vessel model may be derived by combining a total volume variant with an inner volume variant (it will be appreciated that an inner volume model relating to the outer volume of the aorta was discussed in conjunction with Fig. 1 above) by using a geometrical Boolean difference operator. In this case, both sets of parameters characterizing total volume and inner volume, would have to be employed in the configuration of the anatomical model and a particular variant of the inner volume may only be combined with variants of the total volume for which a parameter for the inner volume is less than a parameter for the total volume.

In addition to combination tools, the operators O1 to Oj may also include constructive editing tools. Tools and operators may include, but are not limited to, tools for mesh repair, remeshing, mesh reduction/refinement, Boolean operations (difference, union and intersection) of two or more surface or volume models, trimming/cropping of models, scaling, adding material via offset functions, creation of hollow structures, generation of columns, generation of holes, generation of bridges, deformation of the mesh, offsetting of mesh nodes, rounding of surfaces, smoothing of surfaces, rounding of edges, drilling, extrusion, and/or extraction.

The configuration of the anatomical model may be done manually by a user to being fully automated. For the definition of a configuration, a user interface may be used to collect the intended variants of the different parameters, P1 to Pk, and possibly constructive elements' parameters, CE1 to CEr. Selection restrictions for combination of different parameters as described above may also be integrated in this user interface, e.g., if one parameter is based on a specific variant of another one. In an automated configuration process, the definitions of the anatomical model via a user interface may trigger an automated model generation in a connected process.

Generation of an anatomical model (or a digital tissue model) based on the configuration and combination as described above completes a final step, labelled S8. This anatomical model R9, that may be called a digital or virtual tissue model, may now be further processed as desired. As described above, in embodiments, not all steps S1 to S8 may be employed to carry out the invention as described above. In particular, the present invention may also be carried out starting from an object and generating its variations as described above, without actually generating the object first. This is depicted by dashed arrows in Fig. 2 where, starting from an object, step S4 may be carried out without going through steps S1 to S3, for example, and it will be appreciated that also step S6 is optional.

The processing of the anatomical model described above may be performed in various other programs and software environments. This processing, that may range from manual to fully automated, may be point-based, surface-based and/or volume-based. The range of processes may include, but is not limited to, mesh repair, remeshing, mesh reduction/refinement, Boolean operations (difference, union and intersection) of two or more surface or volume models, trimming/cropping of models, scaling, adding material via offset functions, creation of hollow structures, generation of columns, generation of holes, generation of bridges, deformation of the mesh, offsetting of mesh nodes, rounding of surfaces, smoothing of surfaces, rounding of edges, drilling, extrusion, and/or extraction.

Since anatomical models are typically surface models comprising complex free-form surfaces, corresponding tools for surface editing may be advantageous. Due to the lack of construction elements such as defined edges, faces, or radii, modeling tools for free-form surfaces may be preferred. In particular, the geometric Boolean operators (difference, union and intersection) may be especially well suited for the constructive editing processes. For example, if anatomical models are represented as a set of vertices and corresponding edges between them, it may be relatively simple to perform the operations of union, intersection, and difference between two models by performing the same operations on the individual vertex and edge sets. Such post-processing may be useful, for example, to join anatomical models for the left chamber and the right chamber of a human heart.

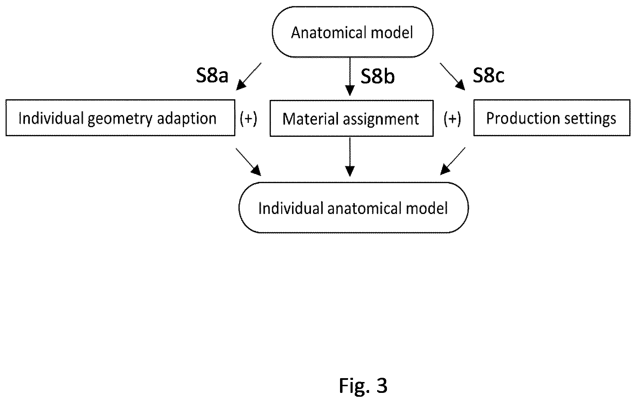

Fig. 3 depicts an embodiment of the present invention where anatomical models generated as described above may be further individualized. In some embodiments, the anatomical models may be individualized by adapting their geometry. This is depicted as the path S8a in Fig. 3. For individual adaptation of the geometry of the anatomical model, the user may individually set additional geometric parameters of the configured model via a user interface, among other things. The user interface may be the same as the one described above, or it may be another user interface provided for this purpose. This may be implemented, among other things, for example, by moving individual mesh nodes of the surface or volume mesh of the anatomical model. By means of conditions stored in the model, further conditional changes may be derived and carried out automatically. For example, if the anatomical model is of a hollow blood vessel and an inner volume of a hollow blood vessel is doubled, a relevant condition might be to keep the ratio of the inner and total volume constant, at 1.5, for example. Then parameters of the volume of the wall, for instance, may be changed automatically to ensure that the ratio of the inner and total volume stays constant at 1.5.

Further, only selected regions may be allowed to be adapted, i.e., not all parameters may be allowed to be varied. Furthermore, the user may scale (if necessary, within restrictions) and crop the anatomical model. The additional adaptation of the configured model may allow the creation of individual models. E.g., a user may crop the model of a configured heart model to only the left side of the heart anatomy and there deform a part of the aortic valve by moving the corresponding mesh nodes. It may be appreciated that in this example, the locations of the mesh nodes may also be comprised by the set of parameters characterizing the digital tissue model.

A further individualization of the anatomical model is depicted as path S8b in Fig. 3. This represents individual assignment of materials and material properties of the model or for subareas of the model for production, in case the digital anatomical model is used to generate a physical model, directly or indirectly as described above. For example, a physical model of a healthy blood vessel may comprise using a more elastic material, than that of a blood vessel for a patient suffering from atherosclerosis.

Yet another individualization of the anatomical model is depicted as path S8c in Fig. 3. This may comprise individual assignment of manufacturing parameters to map specific component properties, e.g., surface roughness. These parameters may include settings for the type of manufacturing, manufacturing direction, tolerances or post-processing option such as coating, abrasive smoothing, coloring, grinding or curing. It will thus be understood that in embodiments of the present invention, the digital model that has been generated may also be used to generate a material model, i.e., a model that is present in the material world. The material model may also be referred to as physical model.

The digital anatomical models may be integrated into a further digital process chain, manufactured in physical form and/or used directly as a digital product. If they are to be manufactured, additive manufacturing may be particularly suitable.

The process of generating a digital anatomical model as described above may be specified, influenced or restricted by application requirements, framework conditions of manufacturing or other influencing factors.

Figs. 4 to 6 depict the creation of a model of a human heart and the connecting major vessels using the database-based anatomical configurator described above.

Fig. 4 depicts an exemplary segmentation of objects, which, directly or indirectly, approximates the geometrical anatomical volumes of a (future) heart model. In a first step (corresponding to S1 in Fig. 2), surface objects 120 (120a, 120b, 120c) may be created from a computer tomographic (CT) image data 110 (110a, 110b, 110c) of a human heart, shown in the top panel of Fig. 4. The panel shows three medical images of the human heart, a sagittal view 110a, a coronal view 110b, and an axial view 110c. These views may be used to generate objects by segmentation as will now be described.

To generate these surface objects, a rough segmentation is done, partially automated, in which voxels are accumulated based on their grayscale values and their regional anatomical location. The resulting segmented objects 120 are depicted in the bottom panel of Fig. 4 and include, e.g., the inner volume of the right atrium 120a, the inner volume of the aorta 120b and the inner volume of the left ventricle 120c.

The same process may be used to generate the inner volumes of the two ventricles, the inner volumes of the two atria as well as the blood volume of the aorta, the vena cava superior, the vena cava inferior and the pulmonary artery. The segmented objects may then be exported, for example, as STL surface meshes. The use of STL meshes may be of advantage in a further manufacturing process, such as an additive manufacturing process.

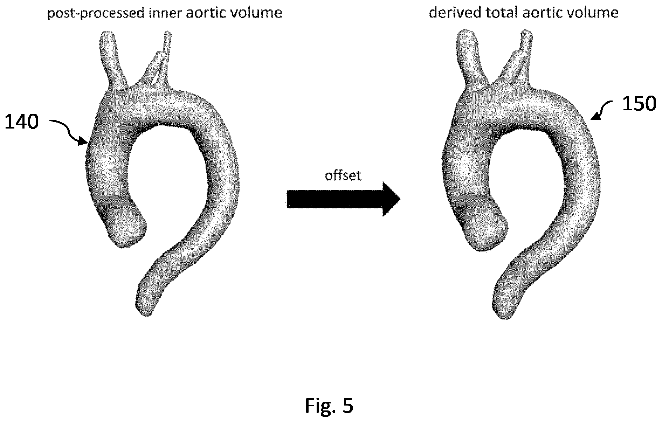

The segmented objects may then be post-processed and/or constructively edited. For example, in Fig. 5, the post-processed inner volume of the aorta 140 may be derived from post-processing the inner volume 120b shown in Fig. 4. Additionally, also new objects can be created by post-processing and/or constructively editing and/or constructively combining other objects. Fig. 5 shows an exemplary derivation of the total aortic volume 150 (i.e., of the outer volume of the aorta) from the post-processed inner volume of the aorta 140 by using the constructive tool of an offset function.

The post-processed inner volume of the aorta 140 may be first parametrized to generate parameters, P1 to Pk. A subset of the parameters P1 to Pk may then be varied.

It will be understood that corresponding considerations also apply independent of whether the object is generated "directly" from medical imaging data (as the inner aortic volume 140) depicted in Fig. 4, or whether the object is generated differently. For example, the object may also be constructively generated, or generated based on other data. The latter is the case for the total aortic volume 150 depicted in Fig. 5. This volume 150 is not "directly" generated from medical imaging data, but is generated based on an original object, i.e., more particularly based on the inner aortic volume 140, e.g., by applying a constant wall thickness to this original object.

It will be understood that for the example of the total volume of the aorta 150 depicted in Fig. 5, different outer diameter variations may be created by, e.g., either applying multiple offsets from the same inner volume of the aorta 140, or by applying offsets from the first total volume of the aorta 150, i.e., by first generating the total volume of the aorta 150 from the inner volume of the aorta 140 and then adding offsets, or by scaling of either the inner volume 140 or the total volume 150. For example, choosing a constant (w.r.t. a position along the aorta) offset from the inner aortic volume 140 for the creation of a model with a constant total volume would result in a constant wall thickness in the future anatomical model, if those parameters were combined. Alternatively, a variant with, e.g., a varied local diameter and cross section may later be used for the configuration of, e.g., an aorta with a pathological thinner partial vascular wall.

Further, possible constructive elements, characterized by the parameters CE1 to CEr, for the heart model may be adapters at the end of the modeled vessel features, for example, to allow connection to a flow simulation system for a manufactured physical heart model.

The different variations of the parameters, P1 to Pk, and constructive elements' parameters, CE1 to CEr, may be stored in a database.

The exemplary derivative of the aorta depicted on the right in Fig. 5 may thus be obtained by choosing a particular configuration of, at least one of, objects characterized by the parameters P1 to Pk.

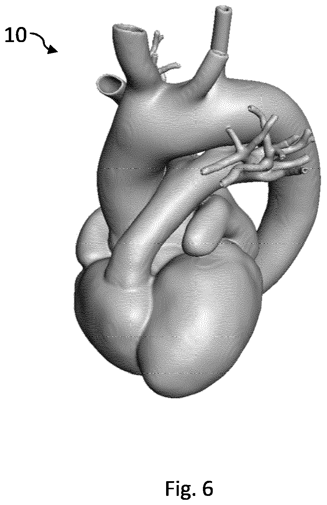

Fig. 6 depicts a configuration of the heart model 10 shown where object variations of different objects and no constructive elements may be chosen, for example, for each of the objects 120 (120a, 120b, 120c) depicted in Fig. 4. For example, the total volume of the aorta 150 as shown in Fig. 5 may be combined with e.g. objects describing the atria (such as the right atrium 120a shown in Fig. 4), and the ventricles (such as the left ventricle 120c shown in Fig. 4) to generate a digital anatomical model 10 for the heart as shown in Fig. 6. A specific order of geometrical Boolean subtraction and addition as well as a subsequent trimming of the ends of the vascular structures and rounding of edges may result in the anatomical heart model 10 depicted in Fig. 6.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

While in the above, preferred embodiments have been described with reference to the accompanying drawings, the skilled person will understand that these embodiments were provided for illustrative purpose only and should by no means be construed to limit the scope of the present invention, which is defined by the claims.

## Claims

1. A system, wherein the system comprises a database,
wherein the system is configured to, for each object (120) of a plurality of objects, vary object parameters and thus generate parameter variations and to generate object variations, and store data relating to the parameter variations in the database,
wherein the system is **characterized in that** it is further configured to generate a plurality of digital tissue models (10), wherein generating each digital tissue model (10) comprises combining object variations of different objects (120),
wherein the system is further configured to store a set of operators, and wherein the object variations are combined using any of the operators in the set of operators,
wherein each object (120) is indicative of a tissue,
wherein the object parameters comprise parameters describing the geometry of the object (120), and
wherein at least one object (120) is a surface in three-dimensional space.

2. The system according to the preceding claim, wherein each object variation is based on a combination of the object parameters and the parameter variations of the respective object (120).

3. The system according to the preceding claim, wherein the system is further configured to
parametrize an object (120) and thus generate the object parameters, and
store the object parameters in the database.

4. The system according to the preceding claim, wherein the system is further configured to generate an object (120) from surface scan data or medical imaging data.

5. The system according to any of the preceding claims, wherein the data relating to the variations comprises the variations, or rules for generating the variations.

6. The system according to any of the preceding claims, wherein the system is configured to store the object (120), the object variations, and/or the plurality of different digital tissue models (10) in the database.

7. The system according to any of the preceding claims, wherein the system is configured to generate a plurality of different material tissue models, wherein each of the material tissue models corresponds to a digital tissue model (10).

8. The system according to any of the preceding claims, wherein the system is configured to apply boundary conditions limiting the varying when varying the object parameters.

9. A method to generate digital tissue models, wherein the method comprises using a system comprising a database, and wherein the method further comprises
for each object (120) of a plurality of objects, varying object parameters and thus generating parameter variations and generating object variations, and storing data relating to the parameter variations in the database,
the method being **characterized in that** it comprises generating a plurality of digital tissue models (10), wherein generating each digital tissue model (10) comprises combining object variations of different objects (120),
wherein the method uses the system according to any of the claims 1 to 8, and
wherein generating a plurality of different tissue models (10) comprises using any of the operators in the set of operators to combine the object variations.

10. The method according to the preceding claim, wherein the method further comprises generating an object (120) from surface scan data or from medical imaging data.

11. The method according to the preceding claim, wherein generating the object (120) from surface scan data or from medical imaging data comprises segmenting images from the surface scan data or the medical imaging data based on a shared property of different parts of the image and either including or excluding parts of the image based on the shared property.

12. The method according to any of the 3 preceding claims, wherein parameterizing the object (120) comprises generating a set of parameters characterizing anatomical features of the object (120), and wherein generating variations comprises choosing values for any of the parameters in the set of parameters characterizing at least one anatomical feature of the object (120).

13. The method according to any of the 4 preceding claims, wherein the method further comprises individualizing any of the plurality of different digital tissue models (10), and wherein individualizing any of the plurality of different digital tissue models (10) comprises adapting the geometry of a digital tissue model (10).

14. A computer program product configured, when run on a digital processing unit of a system, to perform the method according to any of claims 9-13.

## Patentansprüche

1. Ein System, wobei das System eine Datenbank umfasst,
wobei das System so konfiguriert ist, dass es für jedes Objekt (120) einer Mehrzahl von Objekten Objektparameter variiert und dadurch Parametervariationen erzeugt sowie Objektvariationen erzeugt und Daten, die sich auf die Parametervariationen beziehen, in der Datenbank speichert,
wobei das System **dadurch gekennzeichnet ist, dass** es ferner so konfiguriert ist, dass es eine Mehrzahl digitaler Gewebemodelle (10) erzeugt, wobei die Erzeugung jedes digitalen Gewebemodells (10) das Kombinieren von Objektvariationen verschiedener Objekte (120) umfasst,
wobei das System ferner so konfiguriert ist, dass es einen Satz von Operatoren speichert, und wobei die Objektvariationen unter Verwendung eines der Operatoren aus dem Satz der Operatoren kombiniert werden,
wobei jedes Objekt (120) ein Gewebe repräsentiert,
wobei die Objektparameter Parameter umfassen, die die Geometrie des Objekts (120) beschreiben, und
wobei mindestens ein Objekt (120) eine Fläche im dreidimensionalen Raum ist.

2. Das System nach dem vorstehenden Anspruch, wobei jede Objektvariation auf einer Kombination der Objektparameter und der Parametervariationen des jeweiligen Objekts (120) basiert.

3. Das System nach dem vorstehenden Anspruch, wobei das System ferner konfiguriert ist,
ein Objekt (120) zu parametrisieren und dadurch die Objektparameter zu erzeugen, und
die Objektparameter in der Datenbank zu speichern.

4. Das System nach dem vorstehenden Anspruch, wobei das System ferner dazu ausgelegt ist, ein Objekt (120) aus Oberflächenscandaten oder medizinischen Bildgebungsdaten zu erzeugen.

5. System nach einem der vorstehenden Ansprüche, wobei die Daten, die sich auf die Variationen beziehen, die Variationen oder Regeln zur Erzeugung der Variationen umfassen.

6. Das System nach einem der vorstehenden Ansprüche, wobei das System so konfiguriert ist, dass es das Objekt (120), die Objektvariationen und/oder die Mehrzahl verschiedener digitaler Gewebemodelle (10) in der Datenbank speichert.

7. System nach einem der vorstehenden Ansprüche, wobei das System so konfiguriert ist, dass es eine Mehrzahl verschiedener Materialgewebemodelle erzeugt, wobei jedes der Materialgewebemodelle einem digitalen Gewebemodell (10) entspricht.

8. System nach einem der vorstehenden Ansprüche, wobei das System so konfiguriert ist, dass es bei der Variation der Objektparameter Randbedingungen anwendet, die die Variation begrenzen.

9. Verfahren zum Erzeugen digitaler Gewebemodelle, wobei das Verfahren die Verwendung eines Systems umfasst, das eine Datenbank umfasst, und wobei das Verfahren ferner umfasst
für jedes Objekt (120) aus einer Mehrzahl von Objekten das Variieren von Objektparametern und damit das Erzeugen von Parametervariationen sowie das Erzeugen von Objektvariationen und das Speichern von Daten, die sich auf die Parametervariationen beziehen, in der Datenbank,
wobei das Verfahren **dadurch gekennzeichnet ist, dass** es umfasst
das Erzeugen einer Mehrzahl digitaler Gewebemodelle (10), wobei das Erzeugen jedes digitalen Gewebemodells (10) das Kombinieren von Objektvariationen verschiedener Objekte (120) umfasst,
wobei das Verfahren das System gemäß einem der Ansprüche 1 bis 8 verwendet, und
wobei das Erzeugen einer Mehrzahl verschiedener Gewebemodelle (10) die Verwendung eines der Operatoren aus dem Satz der Operatoren zum Kombinieren der Objektvariationen umfasst.

10. Verfahren nach dem vorstehenden Anspruch, wobei das Verfahren ferner das Erzeugen eines Objekts (120) aus Oberflächenscandaten oder aus medizinischen Bildgebungsdaten umfasst.

11. Verfahren nach dem vorstehenden Anspruch, wobei das Erzeugen des Objekts (120) aus Oberflächenscandaten oder aus medizinischen Bildgebungsdaten die Segmentierung von Bildern aus den Oberflächenscandaten oder den medizinischen Bildgebungsdaten auf der Grundlage einer gemeinsamen Eigenschaft verschiedener Bildteile und das Einbeziehen oder Ausschließen von Bildteilen auf der Grundlage der gemeinsamen Eigenschaft umfasst.

12. Verfahren nach einem der drei vorstehenden Ansprüche, wobei das Parametrisieren des Objekts (120) das Erzeugen eines Satzes von Parametern umfasst, die anatomische Merkmale des Objekts (120) charakterisieren, und wobei das Erzeugen von Variationen das Auswählen von Werten für einen der Parameter in dem Satz von Parametern umfasst, die mindestens ein anatomisches Merkmal des Objekts (120) charakterisieren.

13. Verfahren nach einem der vier vorstehenden Ansprüche, wobei das Verfahren ferner das Individualisieren eines der Mehrzahl verschiedener digitaler Gewebemodelle (10) umfasst und wobei das Individualisieren eines der Mehrzahl verschiedener digitaler Gewebemodelle (10) das Anpassen der Geometrie eines digitalen Gewebemodells (10) umfasst.

14. Ein Computerprogrammprodukt, das konfiguriert ist bei Ausführung auf einer digitalen Verarbeitungseinheit eines Systems das Verfahren gemäß einem der Ansprüche 9 bis 13 durchzuführen.

## Revendications

1. Système, dans lequel le système comprend une base de données,
dans lequel le système est configuré pour, pour chaque objet (120) parmi une pluralité d'objets, faire varier des paramètres d'objet et ainsi générer des variations de paramètres et générer des variations d'objet, et stocker des données relatives aux variations de paramètres dans la base de données,
dans lequel le système est **caractérisé par le fait qu'**il est en outre configuré pour générer une pluralité de modèles de tissu numériques (10), la génération de chaque modèle de tissu numérique (10) comprenant la combinaison de variations d'objet de différents objets (120),
dans lequel le système est en outre configuré pour stocker un ensemble d'opérateurs, et dans lequel les variations d'objet sont combinées à l'aide de l'un quelconque des opérateurs dans l'ensemble d'opérateurs,
dans lequel chaque objet (120) indique un tissu,
dans lequel les paramètres d'objet comprennent des paramètres décrivant la géométrie de l'objet (120), et
dans lequel au moins un objet (120) est une surface dans un espace tridimensionnel.

2. Système selon la revendication précédente, dans lequel chaque variation d'objet est basée sur une combinaison des paramètres d'objet et des variations de paramètres de l'objet respectif (120).

3. Système selon la revendication précédente, dans lequel le système est en outre configuré pour
paramétrer un objet (120) et ainsi générer les paramètres d'objet, et
stocker les paramètres d'objet dans la base de données.

4. Système selon la revendication précédente, dans lequel le système est en outre configuré pour générer un objet (120) à partir de données de balayage de surface ou de données d'imagerie médicale.

5. Système selon l'une quelconque des revendications précédentes, dans lequel les données relatives aux variations comprennent les variations, ou des règles pour générer les variations.

6. Système selon l'une quelconque des revendications précédentes, dans lequel le système est configuré pour stocker l'objet (120), les variations d'objet et/ou la pluralité de différents modèles de tissu numériques (10) dans la base de données.

7. Système selon l'une quelconque des revendications précédentes, dans lequel le système est configuré pour générer une pluralité de différents modèles de tissu matériels, chacun desdits modèles de tissu matériels correspondant à un modèle de tissu numérique (10).

8. Système selon l'une quelconque des revendications précédentes, dans lequel le système est configuré pour appliquer des conditions de limite limitant la variation lors de la variation des paramètres d'objet.

9. Procédé de génération de modèles de tissu numériques, dans lequel le procédé comprend l'utilisation d'un système comprenant une base de données, et le procédé comprenant en outre
pour chaque objet (120) parmi une pluralité d'objets, faire varier des paramètres d'objet et ainsi générer des variations de paramètres et générer des variations d'objet, et stocker des données relatives aux variations de paramètres dans la base de données,
le procédé étant **caractérisé par le fait qu'**il comprend
générer une pluralité de modèles de tissu numériques (10), la génération de chaque modèle de tissu numérique (10) comprenant la combinaison de variations d'objet de différents objets (120),
dans lequel le procédé utilise le système selon l'une quelconque des revendications 1 à 8, et
dans lequel la génération d'une pluralité de différents modèles de tissu (10) comprend l'utilisation l'un quelconque des opérateurs dans l'ensemble d'opérateurs pour combiner les variations d'objet.

10. Procédé selon la revendication précédente, dans lequel le procédé comprend en outre la génération d'un objet (120) à partir de données de balayage de surface ou de données d'imagerie médicale.

11. Procédé selon la revendication précédente, dans lequel la génération de l'objet (120) à partir de données de balayage de surface ou de données d'imagerie médicale comprend la segmentation d'images issues des données de balayage de surface ou des données d'imagerie médicale sur la base d'une propriété partagée de différentes parties de l'image et soit l'inclusion, soit l'exclusion de parties de l'image en fonction de la propriété partagée.

12. Procédé selon l'une quelconque des trois revendications précédentes, dans lequel le paramétrage de l'objet (120) comprend la génération d'un ensemble de paramètres caractérisant des caractéristiques anatomiques de l'objet (120), et la génération de variations comprend le choix de valeurs pour l'un quelconque des paramètres dans l'ensemble de paramètres caractérisant au moins une caractéristique anatomique de l'objet (120).

13. Procédé selon l'une quelconque des quatre revendications précédentes, dans lequel le procédé comprend en outre l'individualisation de l'un quelconque de la pluralité de différents modèles de tissu numériques (10), et dans lequel l'individualisation de l'un quelconque de la pluralité de différents modèles de tissu numériques (10) comprend l'adaptation de la géométrie d'un modèle de tissu numérique (10).

14. Produit programme d'ordinateur configuré, lorsqu'il est exécuté sur une unité de traitement numérique d'un système, pour réaliser le procédé selon l'une quelconque des revendications 9 à 13.
